# EUROPEAN PATENT SPECIFICATION

(11) **EP 3 214 074 B1**
(45) Date of publication and mention of the grant of the patent: **18.12.2019**
(21) Application number: 15853900.7
(22) Date of filing: 28.09.2015
(51) Int. Cl.: C07D 231/38, C09B 29/12

(54) **ORGANIC AMINE SALT, PROCESS FOR PRODUCING SAME, AND PROCESS FOR PRODUCING AZO DYE**
ORGANISCHES AMINSALZ, VERFAHREN ZUR HERSTELLUNG DAVON UND VERFAHREN ZUR HERSTELLUNG EINES AZOFARBSTOFFES
SEL D'AMINE ORGANIQUE, SON PROCÉDÉ DE PRODUCTION, ET PROCÉDÉ DE PRODUCTION D'UN COLORANT AZOÏQUE

(30) Priority: 30.10.2014 JP 2014221673
(43) Date of publication of application: 06.09.2017
(73) Proprietor: FUJIFILM Corporation, Tokyo 106-8620 (JP)
(72) Inventor: SATO, Hirotaka, Ashigarakami-gun Kanagawa 258-8577 (JP); KOBAYASHI, Katsumi, Ashigarakami-gun Kanagawa 258-8577 (JP)
(74) Representative: Hoffmann Eitle
(86) International application number: PCT/JP2015/077388
(87) International publication number: WO 2016/067803

(56) References cited:
- WO-A1-2013/130600
- GB-A- 888 167
- JP-A- S5 073 920
- JP-A- H09 127 666
- JP-A- 2006 501 176
- JP-A- 2006 515 282
- JP-A- 2008 540 622
- JP-A- 2010 024 158
- JP-A- 2013 536 866
- US-A1- 2009 181 953

## Description

### BACKGROUND OF THE INVENTION

### 1. Field of the Invention

The present invention relates to an organic amine salt, a method for producing the same, and a method for producing an azo dye.

### 2. Description of the Related Art

Organic amine compounds having an aminopyrazole skeleton are known in the fields of dyeing and the like.

For example, as a dyeing composition by which intense coloration can be attained over a wide range of color tones, and fast coloration can be achieved relatively non-selectively, there is known a dyeing composition including a particular compound having a 4,5-diaminopyrazole skeleton and a coupler (see, for example, JP2003-277247A).

Furthermore, a cationic 4,5-diaminopyrazole derivative having a particular structure is known as a compound useful as a component of a dyeing agent (see, for example, JP2007-511550A).

Similarly, a N-aryl-4,5-diaminopyrazole is known as a compound useful as a component of a dyeing agent (see, for example, JP2006-515282A).

### SUMMARY OF THE INVENTION

However, the organic amine compounds having an aminopyrazole skeleton as described in JP2003-277247A, JP2007-511550A, and JP2006-515282A are likely to be subjected to complicated operations such as concentration and purification (column chromatography or the like), when the compounds are extracted from the reaction liquid after synthesis.

Also, in regard to azo dyes having a pyrazole skeleton, there is a demand for increasing the product yield.

An object of the present disclosure is to provide an organic amine salt having excellent productivity, the organic amine salt being a compound having an aminopyrazole skeleton which can be easily extracted as crystals from the reaction liquid after synthesis without performing complicated operations (concentration, purification, and the like), and to provide a method for producing the organic amine salt.

Another object of the present disclosure is to provide a method for producing an azo dye, by which an azo dye having a pyrazole skeleton can be produced with high yield.

Specific means for achieving the objects described above are as follows.
<1> An organic amine salt, which is a salt of an organic amine compound represented by the following Formula (A) and an acid represented by the following Formula (B): 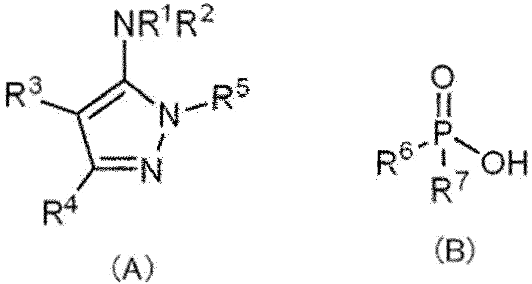
   In Formula (A), each of R¹, R², R³ and R⁴ represents a hydrogen atom and R⁵ represents a methyl group.
   In Formula (B), each of R⁶ and R⁷ represents a hydroxyl group.
<2> A method of producing an organic amine salt, the organic amine salt being the organic amine salt according to <1>, and the method including a step of producing the organic amine salt by subjecting an organic amine compound represented by Formula (A) and an acid represented by Formula (B) to a neutralization reaction.
<3> The method of producing an organic amine salt according to <2>, in which the step of producing an organic amine salt involves subjecting the organic amine compound represented by Formula (A) and the acid represented by Formula (B) to a neutralization reaction by adding the acid represented by Formula (B) to an amine solution containing the organic amine compound represented by Formula (A).
<4> The method of producing an organic amine salt according to <3>, in which the amine solution includes at least one solvent selected from the group consisting of an alcohol and a nitrile-based solvent.
<5> A method of producing an azo dye, the method including a step of producing an azo dye using the organic amine salt according to <1> and an aromatic compound represented by the following Formula (D): 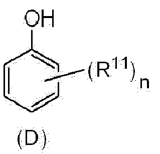
   wherein, in Formula (D), R¹¹ represents a halogen atom, an alkyl group, a halogenated alkyl group, an aromatic group, or a halogenated aromatic group;
   and n represents an integer of from 0 to 4;
   in which the step of producing an azo dye includes: producing a diazonium salt using the organic amine salt; and producing an azo dye by reacting the diazonium salt with the aromatic compound represented by Formula (D).
<6> The method of producing an azo dye according to <5>, wherein the azo dye is an azo dye represented by the following Formula (E): 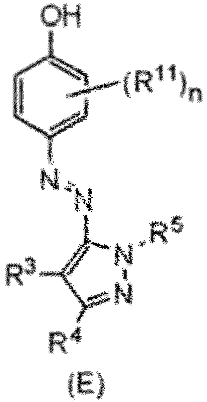
   In Formula (E), R¹¹ represents a halogen atom, an alkyl group, a halogenated alkyl group, an aromatic group, or a halogenated aromatic group; and n represents an integer of from 0 to 4.
   In Formula (E), each of R³ and R⁴ represents a hydrogen atom and R⁵ represents a methyl group.
<7> The method of producing an azo dye according to <6>, in which in Formulae (D) and (E), n represents an integer of from 1 to 4; and R¹¹ represents a halogen atom or a halogenated alkyl group.
<8> The method of producing an organic amine salt according to <7>, in which in Formula (D), at least one R¹¹ is bonded at an ortho-position with respect to the -OH group in Formula (D), and, in Formula (E), at least one R¹¹ is bonded at an ortho-position with respect to the -OH group in Formula (E).
<9> The method of producing an azo dye according to any one of <5> to <8>, in which an amount of the aromatic compound having a hydroxyl group that is used is from 0.8 times to 3.0 times a molar amount of the organic amine salt that is used.
<10> The method of producing an azo dye according to <9>, in which an amount of the aromatic compound having a hydroxyl group that is used is from 1.1 times to 3.0 times a molar amount of the organic amine salt that is used.

According to an aspect of the invention, there are provided an organic amine salt having excellent productivity, the organic amine salt being a compound having an aminopyrazole skeleton which can be easily extracted as crystals from the reaction liquid after synthesis without performing complicated operations (concentration, purification, and the like); and a method for producing the organic amine salt.

According to another aspect of the invention, there is provided a method for producing an azo dye, by which an azo dye having a pyrazole skeleton can be produced with high yield.

### DESCRIPTION OF THE PREFERRED EMBODIMENTS

Hereinafter, the organic amine salt of the present disclosure, a method for producing the same, and a method for producing an azo dye will be described in detail.

According to the present specification, a numerical value range indicated using the symbol "∼" means a range including the numerical values described before and after the symbol "∼" as a lower limit and an upper limit.

### <Organic amine salt>

The organic amine salt of the present disclosure is a salt of an organic amine compound represented by the following Formula (A) and an acid represented by the following Formula (B): 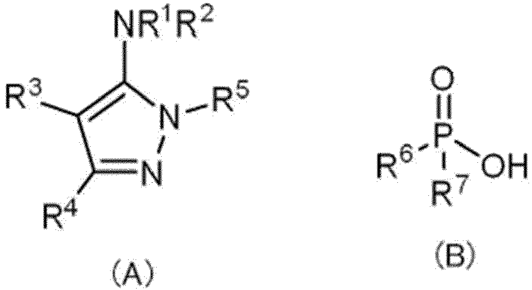
In Formula (A), each of R¹, R², R³ and R⁴ represents a hydrogen atom and R⁵ represents a methyl group.
In Formula (B), each of R⁶ and R⁷ represents a hydroxyl group.

In the present specification, the organic amine compound represented by Formula (A) may also be referred to as "organic amine compound (A)".

Furthermore, in the present specification, the acid represented by Formula (B) may also be referred to as "acid (B)".

Organic amine compounds having an aminopyrazole skeleton are compound that are widely used in the fields of dyeing or the like.

However, regarding an organic amine compound having an aminopyrazole skeleton, when the compound is extracted from the reaction liquid after the synthesis of this organic amine compound, there are occasions in which complicated operations such as concentration and purification (column chromatography or the like) should be performed, and thus productivity is lowered.

In contrast, the organic amine salt of the present disclosure is a compound which can be easily precipitated as crystals without performing complicated operations (concentration, purification, and the like) for the reaction liquid after synthesis, even though the organic amine salt is still a compound (salt) having an aminopyrazole skeleton.

Accordingly, the organic amine salt of the present disclosure is a compound having excellent productivity, which can be easily extracted as crystals from the reaction liquid after synthesis, even without performing complicated operations (concentration, purification, and the like).

Regarding the reason why the organic amine salt of the present disclosure can be easily precipitated as crystals, it is assumed to be because the organic amine salt of the present disclosure is not a simple substance of an organic amine compound, but a salt of an organic amine compound having a particular structure (specifically, organic amine compound (A)) and an acid having a particular structure (specifically, acid (B)).

It is also speculated that it is difficult for a salt of the organic amine compound (A) and an acid other than the acid (B) to be precipitated as crystals from the reaction liquid after synthesis. However, this invention is not intended to be limited by this speculation.

Furthermore, the skeleton of the organic amine compound (A) is not a diaminopyrazole skeleton, which is the skeleton of the compounds described in JP2003-277247A, JP2007-511550A, and JP2006-515282A, but is an aminopyrazole skeleton having only one amino group.

This may also be considered as one of the reasons why the organic amine salt of the present disclosure can be easily precipitated as crystals.

That is, since the skeleton of the organic amine compound (A) is an aminopyrazole skeleton having only one amino group, it is speculated that a salt of the organic amine compound (A) and the acid (B) (that is, the organic amine salt of the present disclosure) can be easily formed, and the salt thus formed can be easily precipitated as crystals. However, this invention is not intended to be limited by this speculation.

Since the organic amine salt of the present disclosure is a compound that can be easily precipitated as crystals, the organic amine salt is a compound that can be produced with high yield, compared to the simple substances of conventional organic amine compounds having an aminopyrazole skeleton.

Furthermore, when an azo dye having a pyrazole skeleton is produced, in a case in which the organic amine salt of the present disclosure is used as a starting material or a synthesis intermediate, the azo dye can be produced with high yield, compared to the case in which the simple substance of a conventional organic amine compound having an aminopyrazole skeleton is used as a starting material or a synthesis intermediate.

As such, the organic amine salt of the present disclosure is useful as a starting material or a synthesis intermediate for the synthesis of an azo dye having a pyrazole skeleton; however, in addition to that, the organic amine salt is also useful as a starting material or a synthesis intermediate for pharmaceutical products, agrochemicals, and the like.

The structure of the organic amine salt of the present disclosure is a structure of a salt formed from a cation obtained as a result of addition of at least one hydrogen atom to the organic amine compound represented by Formula (A) (ammonium ion), and an anion obtained as a result of removal of at least one hydrogen atom from the acid represented by Formula (B).

Here, the position at which a hydrogen atom is added to the organic amine compound represented by Formula (A) is a position on at least one of the at least three nitrogen atoms (N) contained in Formula (A).

The hydrogen atom that is removed from the acid represented by Formula (B) is at least one hydrogen atom included in the at least one hydroxyl group contained in Formula (B).

According to the present specification, indication of the organic amine salt of the present disclosure may be such that the organic amine compound represented by Formula (A) and the acid represented by Formula (B) are described side by side, with the symbol "." disposed therebetween.

Example Compound (1), which is the organic amine salt of the present disclosure, is described as follows:

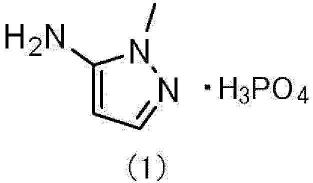

Example Compound (1) is a compound formed from a cation derived from the following Compound A-1 and an anion derived from the following Compound B-1 (phosphoric acid), in which the molar ratio [cation derived from Compound A-1 :anion derived from B-1 (phosphoric acid)] is 1:1.

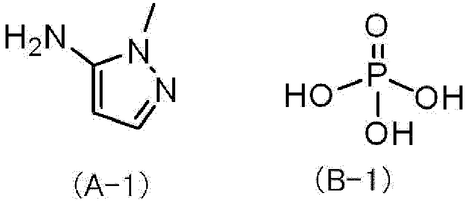

### <Method for producing organic amine salt>

The method for producing an organic amine salt of the present disclosure is a method for producing the organic amine salt of the present disclosure described above, and it is preferable that the method includes a step of subjecting an organic amine compound represented by Formula (A) (organic amine compound (A)) and an acid represented by Formula (B) (acid (B)) to a neutralization reaction, and thereby producing the organic amine salt.

According to the method for producing an organic amine salt of the present disclosure, the organic amine salt of the present disclosure can be easily extracted as crystals without performing complicated operations such as concentration and purification. Therefore, the method for producing an organic amine salt of the present disclosure is a method for producing an organic amine salt with excellent productivity.

Examples of the method of subjecting the organic amine compound and the acid to a neutralization reaction include a method of adding an acid to an amine solution containing the organic amine compound (A), and a method of adding an organic amine compound to an acid solution containing the acid (B).

Here, the concept of "adding the acid (B)" includes not only adding of the acid (B) only, but also adding of an acid solution containing the acid (B). Similarly, the concept of "adding the organic amine compound (A)" includes not only adding of the organic amine compound (A) only, but also adding of an amine solution containing the organic amine compound (A).

Between the two methods described above, the method of adding an acid to an amine solution containing the organic amine compound (A) is preferred, from the viewpoint of producing the organic amine salt efficiently.

Examples of the solvent for the amine solution include an alcohol (methanol, ethanol, isopropanol, t-butanol, ethylene glycol, or the like), an ester (ethyl acetate, isopentyl acetate, or the like), a ketone (y-butyrolactone, acetone, N-methyl-2-pyrrolidone, dimethyl ketone, diisobutyl ketone, cyclopentanone, cyclohexanone, or the like), an ether (diethyl ether, diisopropyl ether, dimethoxyethane, 1,4-dioxane, tetrahydrofuran, anisole, or the like), a glycol ether (ethylene glycol monomethyl ether, ethylene glycol monoethyl ether, ethylene glycol monoethyl ether acetate, or the like), an amide-based solvent (acetamide, N,N-dimethylformamide, N,N-dimethylacetamide, N,N-diethylformamide, or the like), a nitrile-based solvent (acetonitrile, propionitrile, methoxyacetonitrile, or the like), a carbonate-based solvent (dimethyl carbonate, diethyl carbonate, ethyl methyl carbonate, ethylene carbonate, propylene carbonate, or the like), a halogen solvent (methylene chloride, chloroform, dichloromethane, or the like), a hydrocarbon (hexane, cyclohexane, toluene, xylene, or the like), dimethyl sulfoxide, and water.

The solvent for the amine solution may include only one kind thereof, or may include two or more kinds thereof.

It is particularly preferable that the solvent for the amine solution includes at least one selected from the group consisting of alcohols (particularly methanol and ethanol) and nitrile-based solvents (particularly acetonitrile).

The concentration of the organic amine compound (A) in the amine solution is preferably 1% by mass to 100% by mass, more preferably 5% by mass to 50% by mass, and particularly preferably 10% by mass to 30% by mass, with respect to the total amount of the amine solution.

The amine solution can be produced using, for example, a general synthesis method for synthesizing a compound having an aminopyrazole skeleton, such as a cyclization reaction between an acrylonitrile compound and a hydrazine compound (for example, the reaction described in paragraphs 0023 to 0024 of JP2006-515282A).

Examples of the solvent for the acid solution include water, an alcohol (methanol, ethanol, isopropanol, t-butanol, ethylene glycol, or the like), an ester (ethyl acetate, isopentyl acetate, or the like), a ketone (y-butyrolactone, acetone, N-methyl-2-pyrrolidone, dimethyl ketone, diisobutyl ketone, cyclopentanone, cyclohexanone, or the like), an ether (diethyl ether, diisopropyl ether, dimethoxyethane, 1,4-dioxane, tetrahydrofuran, anisole, or the like), a glycol ether (ethylene glycol monomethyl ether, ethylene glycol monoethyl ether, ethylene glycol monoethyl ether acetate, or the like), an amide-based solvent (acetamide, N,N-dimethylformamide, N,N-dimethylacetamide, N,N-diethylformamide, or the like), a nitrile-based solvent (acetonitrile, propionitrile, methoxyacetonitrile, or the like), a carbonate-based solvent (dimethyl carbonate, diethyl carbonate, ethyl methyl carbonate, ethylene carbonate, propylene carbonate, or the like), a halogen solvent (methylene chloride, chloroform, dichloromethane, or the like), a hydrocarbon (hexane, cyclohexane, toluene, xylene, or the like), and dimethyl sulfoxide.

The solvent for the acid solution may include only one kind thereof, or may include two or more kinds thereof.

It is particularly preferable that the solvent for the acid solution includes water.

The concentration of the acid (B) in the acid solution is preferably 1% by mass to 100% by mass, more preferably 50% by mass to 95% by mass, and particularly preferably 80% by mass to 90% by mass, with respect to the total amount of the acid solution.

Furthermore, the reaction temperature for the neutralization reaction between the organic amine compound (A) and the acid (B) is preferably -78°C to 100°C, more preferably 0°C to 50°C, and even more preferably 0°C to 30°C.

### <Method for producing azo dye>

It is preferable that the method for producing an azo dye of the present disclosure includes a step of producing an azo dye using the organic amine salt of the present disclosure.

Since the method for producing an azo dye of the present disclosure produces an azo dye using the organic amine salt of the present disclosure, which can be easily extracted as crystals and can be obtained with high yield, an azo dye having a pyrazole skeleton can be produced with high yield.

Regarding the step of producing an azo dye, an embodiment having a sub-step of producing a diazonium salt using the organic amine salt of the present disclosure; and a sub-step of reacting the diazonium salt with an aromatic compound having a hydroxyl group, and thereby producing an azo dye, is preferred.

Regarding the method for producing a diazonium salt using the organic amine salt of the present disclosure, a known method such as a method of subjecting the organic amine salt to the action of a nitrous acid salt or a nitrous acid ester, can be used.

In regard to the above-described embodiment, it is particularly preferable that the aromatic compound having a hydroxyl group is an aromatic compound represented by the following Formula (D), and the azo dye is an azo dye represented by the following Formula (E). 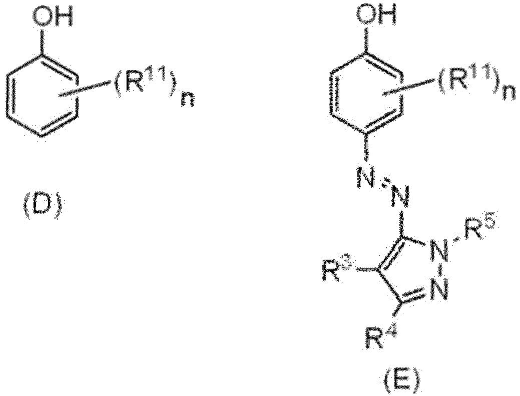
In Formulae (D) and (E), R¹¹ represents a halogen atom, an alkyl group, a halogenated alkyl group, an aromatic group, or a halogenated aromatic group; and n represents an integer from 0 to 4.
In Formula (E), each of R³ and R⁴ represents a hydrogen atom, and R⁵ represents a methyl group.

R³ to R⁵ in Formula (E) have the same meanings as R³ to R⁵ in Formula (A).

Examples of the halogen atom for R¹¹ in Formulae (D) and (E) include a fluorine atom, a chlorine atom, a bromine atom, and an iodine atom.

The halogen atom for R¹¹ in Formulae (D) and (E) is preferably a fluorine atom, a chlorine atom, or a bromine atom, and more preferably a fluorine atom or a chlorine atom.

The alkyl group for R¹¹ in Formulae (D) and (E) may be any of a linear alkyl group, a branched alkyl group, and a cyclic alkyl group (cycloalkyl group).

Examples of the alkyl group for R¹¹ in Formulae (D) and (E) include a methyl group, an ethyl group, a propyl group, an isopropyl group, a butyl group, an isobutyl group, a t-butyl group, a hexyl group, a 2-ethylhexyl group, a dodecyl group, a cyclopropyl group, a cyclopentyl group, a cyclohexyl group, and a 1-adamantyl group.

The alkyl group for R¹¹ in Formulae (D) and (E) is preferably an alkyl group having 1 to 30 carbon atoms, more preferably an alkyl group having 1 to 12 carbon atoms, even more preferably an alkyl group having 1 to 6 carbon atoms, and particularly preferably a methyl group.

The halogenated alkyl group for R¹¹ in Formulae (D) and (E) refers to an alkyl group substituted with at least one halogen atom. In regard to this halogenated alkyl group, examples of the alkyl group to be substituted are a methyl group, an ethyl group, a propyl group, an isopropyl group, a butyl group, an isobutyl group, a t-butyl group, a hexyl group, a 2-ethylhexyl group, a dodecyl group, a cyclopropyl group, a cyclopentyl group, a cyclohexyl group, and a 1-adamantyl group. The alkyl group is preferably an alkyl group having 1 to 30 carbon atoms, more preferably an alkyl group having 1 to 12 carbon atoms, even more preferably an alkyl group having 1 to 6 carbon atoms, and particularly preferably a methyl group.

Examples of the aromatic group for R¹¹ in Formulae (D) and (E) include a phenyl group, a naphthyl group, and a biphenyl group.

The aromatic group for R¹¹ in Formulae (D) and (E) is preferably an aromatic group having 6 to 30 carbon atoms, and particularly preferably a phenyl group.

The halogenated aromatic group for R¹¹ in Formulae (D) and (E) refers to an aromatic group substituted with at least one halogen atom. In regard to this halogenated aromatic group, examples of the aromatic group to be substituted are a phenyl group, a naphthyl group, and a biphenyl group. The aromatic group to be substituted preferably is an aromatic group having 6 to 30 carbon atoms, and particularly preferably a phenyl group.

R¹¹ is preferably a halogen atom or a halogenated alkyl group. A fluorine atom, a chlorine atom, a bromine atom, and a trifluoromethyl group are preferred; a fluorine atom or a chlorine atom is more preferred; and a chlorine atom is particularly preferred.

Furthermore, n is preferably an integer from 0 to 2, more preferably 0 or 1, and particularly preferably 1.

It is also preferable that n represents an integer from 1 to 4, and it is also preferable that n is 1 or 2. In a case in which n represents an integer from 1 to 4, it is preferable that at least one R¹¹ is bonded at the ortho-position with respect to the phenolic hydroxyl group.

Here, the "phenolic hydroxyl group" refers to the -OH group in Formula (D) (namely, the -OH group bonded to the benzene ring in Formula (D)), and the -OH group in Formula (E) (namely, the -OH group bonded to the benzene ring in Formula (E)).

According to the embodiment described above, an amount of the aromatic compound having a hydroxyl group that is used (for example, the aromatic compound represented by Formula (D)) is preferably 0.8 times or more, more preferably 1.1 times or more, and particularly preferably 1.2 times or more, a molar amount of the organic amine salt of the present disclosure that is used.

When the amount of the aromatic compound having a hydroxyl group that is used is 0.8 times or more (particularly 1.1 times or more) the molar amount of the organic amine salt of the present disclosure that is used, the yield of the azo dye is further increased.

The upper limit of the amount of the aromatic compound having a hydroxyl group that is used may be, for example, 3.0 times the molar amount, preferably 2.5 times the molar amount, and more preferably 2.0 times the molar amount.

According to the embodiment described above, the reaction temperature for the reaction between the diazonium salt and the aromatic compound having a hydroxyl group is preferably -5°C to 100°C, more preferably -5°C to 50°C, and particularly preferably -5°C to 35°C.

Furthermore, according to the embodiment described above, the reaction time for the reaction between the diazonium salt and the aromatic compound having a hydroxyl group is preferably 0.5 hours to 24.0 hours, more preferably 0.5 hours to 3.0 hours, and particularly preferably 1.0 hour to 3.0 hours.

After the reaction between the diazonium salt and the aromatic compound having a hydroxyl group, an azo dye can be extracted from the reaction liquid by precipitating the azo dye according to a known method, such as a method of sequentially adding a mixed solvent of water and methanol, and an aqueous solution of sodium acetate to the reaction liquid containing the azo dye produced by the reaction.

### EXAMPLES

Hereinafter, the present invention will be described more specifically by way of Examples; however, the starting materials, the intermediates, and the synthesis routes are not intended to be limited to the following Examples.

In the following description, unless particularly stated otherwise, the "percent (%)" means "percent (%) by mass".

### [Example 1]

### <Synthesis 1 of Example Compound (1)>

Example Compound (1) was synthesized according to the following scheme:

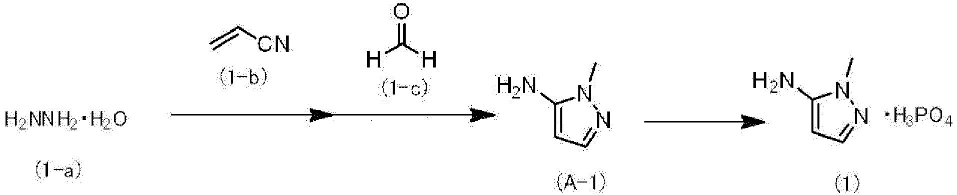

### (Production of Compound (A-1)-containing solution X)

Compound (1-a) (50 g; 1.0 mol) was dissolved in 40 mL of ethanol, and the solution thus obtained was cooled to 0°C. While the cooled solution was stirred, Compound (1-b) (53 g; 1.0 mol) was added dropwise thereto, and then the mixture was stirred for one hour. The solution thus obtained was left to cool naturally, and then ethanol (250 mL) and NaHCO₃ (2.5 g) were added thereto, and Compound (1-c) (31 g; 1.0 mol) was further added thereto. The solution thus obtained was warmed to an internal temperature of 45°C, and was stirred for 30 minutes. The solution obtained after stirring for 30 minutes was cooled to 0°C, and then NaOEt (sodium ethoxide) (3 g) was added thereto. The solution thus obtained was heated to an internal temperature of 85 °C, was stirred for 3 hours, and then was left to cool naturally.

Thus, a solution containing Compound (A-1) (Compound (A-1)-containing solution X) was obtained.

### (Synthesis of Example Compound (1))

900 mL of methanol was added to the Compound (A-1)-containing solution X. While the solution thus obtained was stirred at an internal temperature of 5°C, a 85% aqueous solution of H₃PO₄ (120 g; 1.05 mol as the amount of H₃PO₄) was added dropwise thereto, and crystals were precipitated. The crystals thus precipitated were collected by filtration, and pale yellow to white crystals (111 g) of the Example Compound (1) were obtained (yield 57%). The yield as used herein is a yield based on the amount of the raw material Compound (1-a) (the same also applies to Example 2 that will be described below).

### The structure of the Example Compound (1) was checked by ion chromatography.

As a result, it was confirmed that the Example Compound (1) was a compound in which the molar ratio [cation derived from Compound A-1:anion derived from B-1 (phosphoric acid)] was 1:1.

### [Example 2]

The operations were carried out in the same manner as in Example 1, except that the 900 mL of methanol added to the Compound (A-1)-containing solution X in Example 1 was changed to 900 mL of acetonitrile. As a result, pale yellow to white crystals (113 g) of Example Compound (1) were obtained (yield 58%).

### [Comparative Example 1]

The operations were carried out in the same manner as in Example 1, except that the 85% aqueous solution of H₃PO₄ (120 g) used in Example 1 was changed to a 4 N aqueous solution of HCl in an amount including HCl (1.05 mol). However, crystals were not precipitated, and the Example Compound (1) could not be obtained.

### [Comparative Example 2]

The operations were carried out in the same manner as in Example 1, except that the 85% aqueous solution of H₃PO₄ (120 g) used in Example 1 was changed to a 85% aqueous solution of H₂SO₄ in an amount including H₂SO₄ (1.05 mol). However, crystals were not precipitated, and the Example Compound (1) could not be obtained.

The yield and the like for Examples 1 and 2, and Comparative Examples 1 and 2 are summarized in the following Table 1.

**[Table 1]**

| | Acid added dropwise | Main solvent | Yield |
|---|---|---|---|
| Example 1 | H₃PO₄ | Methanol | 57% |
| Example 2 | H₃PO₄ | Acetonitrile | 58% |
| Comparative Example 1 | HCl | Methanol | 0% |
| Comparative Example 2 | H₂SO₄ | Methanol | 0% |

As shown in Table 1, in Examples 1 and 2 in which phosphoric acid (H₃PO₄) was added dropwise to the Compound (A-1)-containing solution X, the Example Compound (1) could be extracted as crystals with high yield.

In contrast, in Comparative Example 1 in which hydrochloric acid (HCl) was added dropwise to the Compound (A-1)-containing solution X, and in Comparative Example 2 in which sulfuric acid (H₂SO₄) was added dropwise to the Compound (A-1)-containing solution X, the Example Compound (1) could not be extracted as crystals.

Thus, it was confirmed that the Example Compound (1) was an organic amine salt having excellent productivity, which can be extracted as crystals with high yield without performing complicated operations for concentration or purification, even though the Example Compound (1) is a compound having an aminopyrazole skeleton.

### [Example 3]

### <Synthesis 2 of Example Compound (1)>

Example Compound (1) was synthesized according to the following scheme.

This Example 3 is an example in which the method for producing a solution containing Compound (A-1) is different from that of Example 1.

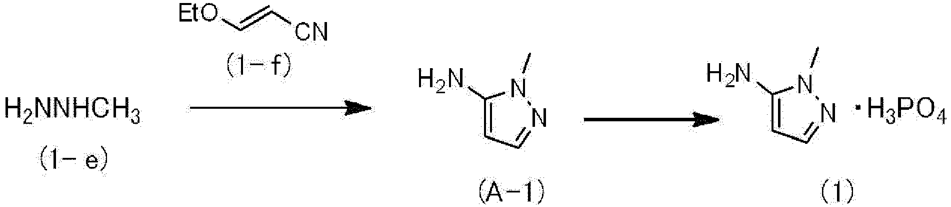

### (Production of Compound (A-1)-containing solution Y)

Compound (1-e) (46 g; 1.0 mol) was dissolved in 500 mL of ethanol, and the solution thus obtained was cooled to an internal temperature of 5°C. While the cooled solution was stirred, Compound (1-f) (97 g; 1.0 mol) was added dropwise thereto. The solution thus obtained was heated to an internal temperature of 95°C, was stirred for 8 hours, and then was left to cool naturally.

Thus, a solution containing Compound (A-1) (Compound (A-1)-containing solution Y) was obtained.

### (Synthesis of Example Compound (1))

900 mL of methanol was added to the Compound (A-1)-containing solution Y. While the solution thus obtained was stirred at an internal temperature of 5°C, a 85% aqueous solution of H₃PO₄ (120 g) was added dropwise thereto, and crystals were precipitated. The crystals thus precipitated were collected by filtration, and pale yellow to white crystals of Example Compound (1) (136 g) were obtained (yield 70%). The yield as used herein is a yield based on the amount of the raw material Compound (1-e).

The structure of the Example Compound (1) was checked in the same manner as in Example 1.

Thus, even in a case in which the method for producing the Compound (A-1)-containing solution was different from that of Example 1, similarly to Example 1, the Example Compound (1) could be extracted as crystals without performing complicated operations for concentration or purification.

### [Example 101]

### <Synthesis of azo dye (E-1)>

Azo dye (E-1) was synthesized according to the following scheme:

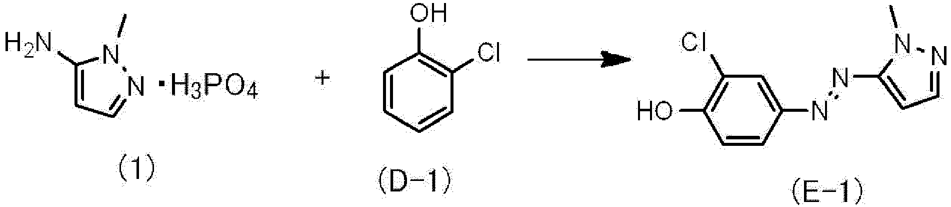

A solution (a) in which Example Compound (1) (1.0 mol) was dissolved in a mixed solution of propionic acid (60 mL), a 85% aqueous solution of H₃PO₄ (50 mL), and acetic acid (50 mL), was produced.

Furthermore, sulfuric acid (230 mL) was cooled to an internal temperature of 5°C, and while the mixture was stirred, NaNO₃ (69 g in total) was added thereto in divided portions. Thus, a solution (b) was obtained. The solution (b) thus obtained was heated to an internal temperature of 70°C, was stirred for 2 hours, and then was cooled to 0°C.

Next, 40 mL of sulfuric acid was added to the solution (b) that had been cooled to 0°C, and the solution (a) produced as described above was added dropwise thereto. Thus, a diazo liquid containing a diazonium salt derived from the Example Compound (1) was obtained.

The diazo liquid thus obtained was added dropwise to a methanol (300 mL) solution of Compound (D-1) (1.0 mol) at an internal temperature of 0°C while the solution was stirred. The solution thus obtained was stirred for 2 hours, and then was diluted with water and methanol.

An aqueous solution of sodium acetate was added to the diluted solution, and the mixture was stirred for one hour. Then, crystals were precipitated. The crystals thus precipitated were collected by filtration, and thereby 70 g of azo dye (E-1) was obtained (yield 30%). The yield as used herein is a yield based on the amount of the Example Compound (1) (the same also applies to Example 102).

### [Example 102]

The operations were carried out in the same manner as in Example 101, except that the amount of Example Compound (1) (1.0 mol) used in Example 101 was changed to 0.7 mol, and thus 110 g of azo dye (E-1) was obtained (yield 66%).

### [Comparative Example 101]

A Compound (A-1)-containing solution X was produced byte the method disclosed in Example 1.

The Compound (A-1)-containing solution X was concentrated, and then acetonitrile and saturated saline were added thereto. Thus, the mixture was partitioned into an aqueous layer and an organic layer. The organic layer was concentrated, and thereby Compound (A-1) (69 g) was obtained as a black solid (crude yield 69%).

The operations were carried out in the same manner as in Example 101, except that the Example Compound (1) (1.0 mol) used in Example 101 was changed to 1.0 mol of the aforementioned black solid in an amount in terms of Compound (A-1), and thus 50 g of the azo dye (E-1) was obtained (yield 21%). The yield as used herein is a yield based on the amount of Compound (A-1) (the same also applies to Example 102).

The yield and the like for Examples 101 and 102, and Comparative Example 101 are summarized in the following Table 2.

**[Table 2]**

| | Raw material | Mol number of raw material | Yield |
|---|---|---|---|
| Example 101 | (1) | 1.0 | 30% |
| Example 102 | (1) | 0.7 | 66% |
| Comparative Example 101 | (A-1) | 1.0 | 21% |

As shown in Table 2, in Examples 101 and 102 in which Example Compound (1) as a salt of a particular organic amine and a particular acid was used as a raw material, azo dyes could be synthesized with high yield, compared to Comparative Example 101 in which Compound (A-1) that was a particular organic amine but was not a salt was used.

## Claims

1. An organic amine salt, which is a salt of an organic amine compound represented by the following Formula (A) and an acid represented by the following Formula (B): 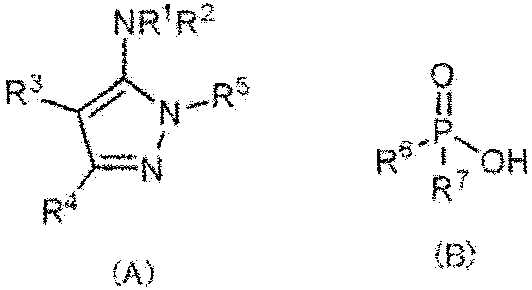
wherein, in Formula (A), each of R¹, R², R³ and R⁴ represents a hydrogen atom and R⁵ represents a methyl group;
wherein, in Formula (B), each of R⁶ and R⁷ represents a hydroxyl group.

2. A method of producing an organic amine salt, the organic amine salt being the organic amine salt according to claim 1, and the method comprising a step of producing the organic amine salt by subjecting the organic amine compound represented by Formula (A) and the acid represented by Formula (B) to a neutralization reaction.

3. The method of producing an organic amine salt according to claim 2, wherein the step of producing the organic amine salt comprises subjecting the organic amine compound represented by Formula (A) and the acid represented by Formula (B) to a neutralization reaction by adding the acid represented by Formula (B) to an amine solution containing the organic amine compound represented by Formula (A).

4. The method of producing an organic amine salt according to claim 3, wherein the amine solution comprises at least one solvent selected from the group consisting of an alcohol and a nitrile-based solvent.

5. A method of producing an azo dye, the method comprising a step of producing an azo dye using the organic amine salt according to claim 1 and an aromatic compound represented by the following Formula (D); 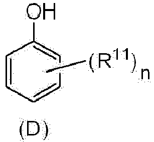
wherein, in Formula (D), R¹¹ represents a halogen atom, an alkyl group, a halogenated alkyl group, an aromatic group, or a halogenated aromatic group;
and n represents an integer of from 0 to 4;
wherein the step of producing the azo dye includes:
producing a diazonium salt using the organic amine salt; and
producing an azo dye by reacting the diazonium salt with the aromatic compound represented by Formula (D).

6. The method of producing an azo dye according to claim 5, wherein the azo dye is an azo dye represented by the following Formula (E): 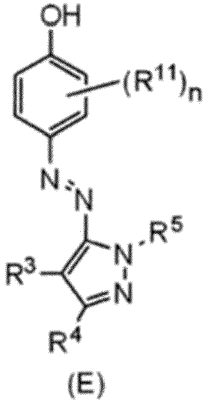 wherein, in Formula (E), R¹¹ represents a halogen atom, an alkyl group, a halogenated alkyl group, an aromatic group, or a halogenated aromatic group; n represents an integer of from 0 to 4; each of R³ and R⁴ represents a hydrogen atom and R⁵ represents a methyl group.

7. The method of producing an azo dye according to claim 6, wherein, in Formulae (D) and (E), n represents an integer of from 1 to 4, and R¹¹ represents a halogen atom or a halogenated alkyl group.

8. The method of producing an azo dye according to claim 7, wherein in Formula (D), at least one R¹¹ is bonded at an ortho-position with respect to the -OH group in Formula (D), and, in Formula (E), at least one R¹¹ is bonded at an ortho-position with respect to the -OH group in Formula (E).

9. The method of producing an azo dye according to any one of claims 5 to 8, wherein an amount of the aromatic compound represented by Formula (D) that is used is from 0.8 times to 3.0 times a molar amount of the organic amine salt that is used.

10. The method of producing an azo dye according to claim 9, wherein an amount of the aromatic compound represented by Formula (D) that is used is from 1.1 times to 3.0 times a molar amount of the organic amine salt that is used.

## Patentansprüche

1. Organisches Aminsalz, welches ein Salz einer durch die folgende Formel (A) dargestellten organischen Aminverbindung und einer durch die folgende Formel (B) dargestellten Säure ist: 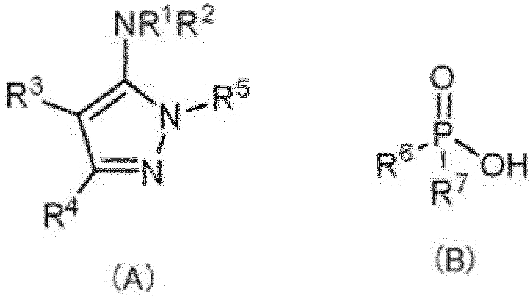
worin in Formel (A) jedes von R¹, R², R³ und R⁴ ein Wasserstoffatom darstellt und R⁵ eine Methylgruppe darstellt;
worin in Formel (B) jedes von R⁶ und R⁷ eine Hydroxylgruppe darstellt.

2. Verfahren zur Herstellung eines organischen Aminsalzes, wobei das organische Aminsalz das organische Aminsalz gemäß Anspruch 1 ist und das Verfahren einen Schritt zur Herstellung des organischen Aminsalzes umfasst, indem die durch die Formel (A) dargestellte organische Aminverbindung und die durch die Formel (B) dargestellte Säure einer Neutralisationsreaktion unterzogen werden.

3. Verfahren zur Herstellung eines organischen Aminsalzes gemäß Anspruch 2, worin der Schritt zur Herstellung des organischen Aminsalzes umfasst, dass die durch die Formel (A) dargestellte organische Aminverbindung und die durch die Formel (B) dargestellte Säure einer Neutralisationsreaktion unterzogen werden, indem die durch die Formel (B) dargestellte Säure zu einer Aminlösung zugegeben wird, die die durch die Formel (A) dargestellte organische Aminverbindung enthält.

4. Verfahren zur Herstellung eines organischen Aminsalzes gemäß Anspruch 3, worin die Aminlösung zumindest ein Lösungsmittel umfasst, ausgewählt aus der Gruppe bestehend aus einem Alkohol und einem Nitril-basierten Lösungsmittel.

5. Verfahren zur Herstellung eines Azofarbstoffes, wobei das Verfahren einen Schritt zur Herstellung eines Azofarbstoffes unter Verwendung des organischen Aminsalzes gemäß Anspruch 1 und einer aromatischen Verbindung, dargestellt durch die folgende Formel (D), umfasst: 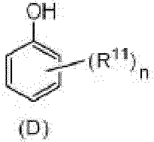
worin in der Formel (D) R¹¹ ein Halogenatom, eine Alkylgruppe, eine halogenierte Alkylgruppe, eine aromatische Gruppe oder eine halogenierte aromatische Gruppe darstellt; und n eine ganze Zahl von 0 bis 4 darstellt;
worin der Schritt zur Herstellung des Azofarbstoffs umfasst:
Herstellen eines Diazoniumsalzes unter Verwendung des organischen Aminsalzes; und
Herstellen eines Azofarbstoffes durch Umsetzen des Diazoniumsalzes mit der durch die Formel (D) dargestellten aromatischen Verbindung.

6. Verfahren zur Herstellung eines Azofarbstoffes gemäß Anspruch 5, worin der Azofarbstoff ein durch die folgende Formel (E) dargestellte Azofarbstoff ist: 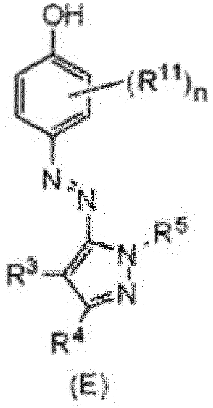 worin in der Formel (E) R¹¹ ein Halogenatom, eine Alkylgruppe, eine halogenierte Alkylgruppe, eine aromatische Gruppe oder eine halogenierte aromatische Gruppe darstellt; n eine ganze Zahl von 0 bis 4 darstellt; jedes von R³ und R⁴ ein Wasserstoffatom darstellt und R⁵ eine Methylgruppe darstellt.

7. Verfahren zur Herstellung eines Azofarbstoffes gemäß Anspruch 6, worin in den Formeln (D) und (E) n eine ganze Zahl von 1 bis 4 darstellt und R¹¹ ein Halogenatom oder eine halogenierte Alkylgruppe darstellt.

8. Verfahren zur Herstellung eines Azofarbstoffes gemäß Anspruch 7, worin in Formel (D) zumindest ein R¹¹ an einer ortho-Position in Bezug auf die -OH-Gruppe in Formel (D) gebunden ist, und in Formel (E) zumindest ein R¹¹ an einer ortho-Position in Bezug auf die -OH-Gruppe in Formel (E) gebunden ist.

9. Verfahren zur Herstellung eines Azofarbstoffes gemäß irgendeinem der Ansprüche 5 bis 8, worin die Menge der durch die Formel (D) dargestellten verwendeten aromatischen Verbindung vom 0,8-fachen bis 3,0-fachen der molaren Menge des verwendeten organischen Aminsalzes beträgt.

10. Verfahren zur Herstellung eines Azofarbstoffes gemäß Anspruch 9, worin die Menge der durch die Formel (D) dargestellten verwendeten aromatischen Verbindung vom 1,1-fachen bis 3,0-fachen der molaren Menge des verwendeten organischen Aminsalzes beträgt.

## Revendications

1. Sel d'amine organique, qui est un sel d'un composé d'amine organique représenté par la formule (A) suivante et d'un acide représenté par la formule (B) suivante : 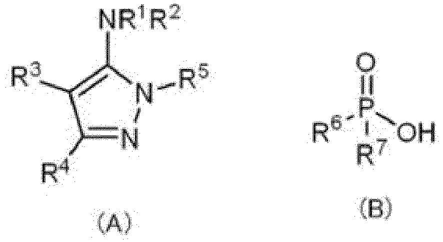
dans laquelle, dans la formule (A), chacun de R¹, R², R³ et R⁴ représente un atome d'hydrogène et R⁵ représente un groupe méthyle ;
dans laquelle, dans la formule (B), chacun de R⁶ et R⁷ représente un groupe hydroxyle.

2. Procédé de production d'un sel d'amine organique, le sel d'amine organique étant le sel d'amine organique selon la revendication 1, et le procédé comprenant une étape de production du sel d'amine organique par la soumission du composé d'amine organique représenté par la formule (A) et de l'acide représenté par la formule (B) à une réaction de neutralisation.

3. Procédé de production d'un sel d'amine organique selon la revendication 2, dans lequel l'étape de production du sel d'amine organique comprend la soumission du composé d'amine organique représenté par la formule (A) et de l'acide représenté par la formule (B) à une réaction de neutralisation par l'ajout de l'acide représenté par la formule (B) à une solution d'amine contenant le composé d'amine organique représenté par la formule (A).

4. Procédé de production d'un sel d'amine organique selon la revendication 3, dans lequel la solution d'amine comprend au moins un solvant sélectionné dans le groupe consistant en un alcool et un solvant à base de nitrile.

5. Procédé de production d'un colorant azoïque, le procédé comprenant une étape de production d'un colorant azoïque en utilisant le sel d'amine organique selon la revendication 1 et un composé aromatique représenté par la formule (D) suivante : 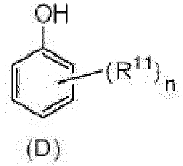
dans laquelle, dans la formule (D), R¹¹ représente un atome d'halogène, un groupe alkyle, un groupe alkyle halogéné, un groupe aromatique, ou un groupe aromatique halogéné ; et n représente un nombre entier allant de 0 à 4 ;
dans lequel l'étape de production du colorant azoïque inclut :
la production d'un sel de diazonium en utilisant le sel d'amine organique ; et
la production d'un colorant azoïque par la réaction du sel de diazonium avec le composé aromatique représenté par la formule (D).

6. Procédé de production d'un colorant azoïque selon la revendication 5, dans lequel le colorant azoïque est un colorant azoïque représenté par la formule (E) suivante : 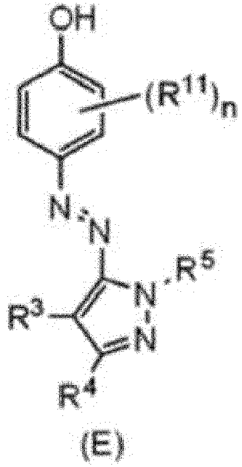 dans laquelle, dans la formule (E), R¹¹ représente un atome d'halogène, un groupe alkyle, un groupe alkyle halogéné, un groupe aromatique, ou un groupe aromatique halogéné ; n représente un nombre entier allant de 0 à 4 ; chacun de R³ et R⁴ représente un atome d'hydrogène et R⁵ représente un groupe méthyle.

7. Procédé de production d'un colorant azoïque selon la revendication 6, dans lequel, dans les formules (D) et (E), n représente un nombre entier allant de 1 à 4, et R¹¹ représente un atome d'halogène ou un groupe alkyle halogéné.

8. Procédé de production d'un colorant azoïque selon la revendication 7, dans lequel dans la formule (D), au moins un R¹¹ est lié à une position ortho par rapport au groupe -OH dans la formule (D), et, dans la formule (E), au moins un R¹¹ est lié à une position ortho par rapport au groupe -OH dans la formule (E).

9. Procédé de production d'un colorant azoïque selon l'une quelconque des revendications 5 à 8, dans lequel une quantité du composé aromatique représenté par formule (D) qui est utilisée est de 0,8 fois à 3,0 fois une quantité molaire du sel d'amine organique qui est utilisée.

10. Procédé de production d'un colorant azoïque selon la revendication 9, dans lequel une quantité du composé aromatique représenté par la formule (D) qui est utilisée est de 1,1 fois à 3,0 fois une quantité molaire du sel d'amine organique qui est utilisée.
